# EUROPEAN PATENT APPLICATION

(11) **EP 3 903 685 A1**
(43) Date of publication of application: **03.11.2021**
(21) Application number: 20171699.0
(22) Date of filing: 28.04.2020
(51) Int. Cl.: A61B 8/12, A61B 8/00

(54) **CATHETER OR GUIDEWIRE SYSTEMS WITH INTEGRATED ULTRASOUND IMAGING**

(71) Applicant: Koninklijke Philips N.V., 5656 AG Eindhoven (NL)
(72) Inventor: VAN RENS, Antonia Cornelia, 5656 AE Eindhoven (NL); DOUGLAS, Alexander Ulrich, 5656 AE Eindhoven (NL); MOUNAIM, Amine, 5656 AE Eindhoven (NL)
(74) Representative: Philips Intellectual Property & Standards

(57) **Abstract**

A catheter or guidewire system, comprises a console unit and a tip comprising sensors and/or actuators. The console unit has a first bridge circuit for converting between a first communication protocol based on a two-wire single-ended open drain architecture and a second communication protocol based on two differential wire pairs. The tip comprises a second bridge circuit for converting between the first and second communication protocols and for communicating with the sensors and/or actuators using the first communication protocol. This enables a four wire high-speed control interface. The interface may for example use the well-known I2C interface standard.

## Description

### FIELD OF THE INVENTION

The invention relates to ultrasound imaging catheters or guidewires.

### BACKGROUND OF THE INVENTION

Diagnosis and treatment in healthcare procedures can be improved by integrating imaging and sensing devices at the tip of interventional instruments - such as catheters and guidewires. The very limited size of these instruments poses an important challenge on integration of the required imaging/sensing functionality.

An IVUS (intra vascular ultrasound) phased array catheter integrates multiple ASIC dies in the tip of a catheter to control an integrated transducer array. An example of an existing product is the Eagle Eye Platinum (EEP) catheter that holds five ASIC dies that are placed in the tip of the catheter to control 64 transducer elements that are placed as a caterpillar around the circumference of the device. To improve image quality and deliverability, next generation phased array IVUS catheters will include even more transducer elements, operated at higher ultrasound frequencies and integrated in a smaller diameter catheter.

To enable a catheter to be optimized for deliverability, it has a small catheter diameter as well as a short stiff tip length. The small diameter means the number of system wires has to be minimized as well as their diameter. This has a strong impact on the electrical wire characteristics, in particular for high frequencies. To guarantee signal integrity, it is known to transfer the transducer echo signals in a differential way using twisted pair system wires and characteristic impedance matching. The required image framerate is preferably high to allow fast pull-back. Fast (re)programming of the ASIC dies is required to enable this.

An I2C (2-wire) interface is an attractive catheter control interface as it is well-known and as it requires only two control wires for bi-directional communication independent of the number of connected devices. However, the interface is too slow due to the usage of open-drain circuitry and pull-up resistors, in particular when loaded heavily. Programming speed can be increased by using low-value pull up resistors but this is at the expense of high power dissipation which is not acceptable in the case of a catheter interface.

There is a need for a high-speed low power, low wire-count, control interface between the system console and the sensor or actuator dies that are placed at the tip of a catheter.

### SUMMARY OF THE INVENTION

The invention is defined by the claims.

According to examples in accordance with an aspect of the invention, there is provided a catheter or guidewire system, comprising:
a console unit;
a tip comprising sensors and/or actuators; and
a catheter shaft or guidewire between the console unit and the tip,
wherein the console unit comprises a first bridge circuit for converting between a first communication protocol based on a two-wire single-ended open drain architecture and a second communication protocol based on two differential wire pairs, with one differential wire pair for each of the two wires of the first communication protocol,
wherein the tip comprises a second bridge circuit for converting between the first and second communication protocols and for communicating with the sensors and/or actuators using the first communication protocol,
wherein the catheter shaft or guidewire comprises two differential wire pairs connected between the first and second bridge circuits for communicating using the second communication protocol,
and wherein each bridge circuit is terminated to match the characteristic impedance of the differential wire pairs and wherein the differential signals are superimposed on a common mode voltage.

This enables a four wire high-speed control interface. The interface may for example use the well-known I2C interface standard. The long catheter shaft or guidewire, for example up to 3m in length, uses two differential wire pairs, for example one for data and one for clock signals. The local data transmission, within the tip or the console unit, uses an open drain communication protocol. The short distances within the tip and the console unit avoid issues with the speed of the protocol.

In particular, the length of the interconnects at the tip and at the console are relatively short and power dissipation is not of significant relevance. The lengths of the interconnects are for example up to 10cm, and typically less than 1cm, so the local interconnects do not behave as a transmission line (due to the frequency of up to 50MHz), but can instead be considered to be a lumped capacitive load. Due to the short distances, the capacitive load of the open drain output delivered by the first communication protocol can remain low as well. The capacitive load related to a 10 cm interconnect may be compensated by choosing lower pull up resistor values.

The second protocol for example uses impedance-matched differential wire pairs to achieve high data rates over long differential wire pairs. The termination impedances are thus matched to the wire impedance.

The termination of each bridge circuit for example comprises a respective series pair of termination impedances between the wires of each differential wire pair, wherein the junction between the pair of termination impedances is connected to the common mode voltage.

The communication between the first and second bridge circuits for example comprises a half duplex communication. Data communication is thus possible in two directions using the same wires, but not at the same time.

Each differential wire pair for example has a same common mode voltage. Thus, a common mode voltage terminal may connect to both differential wire pairs, for example at a node between a pair of termination impedances.

The tip for example comprises an ultrasound imaging device. Thus, the system is an ultrasound imaging catheter or guidewire.

The tip may comprise an array of ultrasound transducer elements formed as a plurality of ultrasound transducer sub-arrays, each sub-array having an associated control circuit, wherein the second bridge circuit communicates with each of the control circuits using the first communication protocol. There may for example be separate ASICs for each sub-array of transducers. The sub-arrays of transducers for example form an annulus around the catheter or guidewire tip.

The console unit may comprise a respective pull up resistor for each of the two wires for communicating using the first communication protocol, and the tip comprises a respective pull up resistor for each of the two wires for communicating using the first communication protocol. This is a standard open drain configuration.

The first communication protocol for example uses a pair of rail voltages with a voltage difference in the range 1.8V to 5V, and the second communication protocol uses differential voltages of below 1.8V. Thus, the long range communication is a low voltage differential scheme, and the communication in the tip and console unit is a higher voltage scheme.

The second communication protocol may use different voltage amplitudes for the tip and for the console unit. This allows dissipation to be minimized in the catheter or guidewire and allows for easy signal detection in the catheter or guidewire tip. Usage of different signaling amplitudes is also attractive as it allows half-duplex communication and prevents communication collisions as the master (console) can always overrule the signaling of the slave (tip).

The first communication protocol is for example the I²C communication protocol.

Each bridge circuit may comprise a respective pair of back-to-back amplifier circuits between each one of the wires for communicating using the first communication protocol and each associated one of the differential wire pairs. These amplifier circuits provide conversion between voltage of the first communication protocol and voltage of the second communication protocol.

The console unit for example comprises a circuit for setting the common mode voltage.

The second communication protocol for example has a data rate above 10MHz, and:
uses a zero differential voltage to encode an idling mode; and/or
uses a zero DC signaling scheme.

A first one of the two differential wire pairs is preferably a clock signal wire pair and a second one of the two differential wire pairs is a data signal wire pair. These are derived from corresponding data and clock signal lines of the first communication protocol.

Alternatives may be possible. For example, clock-signal could be replaced by a Manchester encoded clock and data signal (in one signal) driven from the console side and the data signal could be dedicated for the data from tip to the console side.

The data rate of the first and second protocols is the same, although different communication speeds may be used for communication in the two directions (master to slave i.e. write and slave to master i.e. read).

Each bridge circuit may comprise a controller and a memory, wherein the memory stores address information of modules that are connected to the respective bridge circuit using the first communication protocol.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows in schematic form a catheter or guidewire system;
Figure 2 shows the system of Figure 1 in more detail for an ultrasound system;
Figure 3 shows a block diagram of the bridge circuit functionality;
Figure 4 shows a pair of connected bridge circuits of the type shown in Figure 3; and
Figure 5 shows one possible signaling scheme.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a catheter or guidewire system, comprising a console unit and a tip comprising sensors and/or actuators. The console unit has a first bridge circuit for converting between a first communication protocol based on a two-wire single-ended open drain architecture and a second communication protocol based on two differential wire pairs. The tip comprises a second bridge circuit for converting between the first and second communication protocols and for communicating with the sensors and/or actuators using the first communication protocol. This enables a four wire high-speed control interface. The interface may for example use the well-known I2C interface standard.

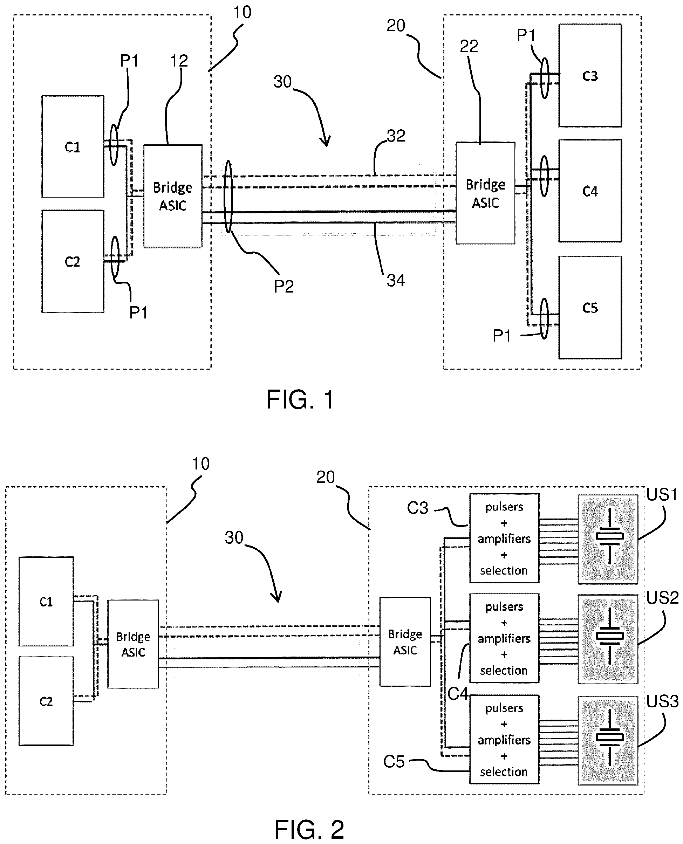

Figure 1 shows in schematic form a catheter or guidewire system, comprising a console unit 10 having control circuits C1 and C2 and a tip 20 comprising sensors and/or actuators formed as, or controlled by, circuits C3, C4 and C5. These circuits are for example ASICs or general purpose chips.

The example of a catheter system will be described, in which a catheter shaft 30 extends between the console unit 10 and the tip 20. The same system may be applied to a guidewire.

The console unit comprises a first bridge circuit 12 for converting between a first communication protocol P1 based on a two-wire single-ended open drain architecture and a second communication protocol P2 based on two differential wire pairs, with one differential wire pair for each of the two wires of the first communication protocol.

The ultrasound tip comprises a second bridge circuit 22 for converting between the first and second communication protocols PI, P2 and for communicating with the sensors and/or actuators using the first communication protocol P1.

The catheter shaft 30 comprises two differential wire pairs 32, 34 connected between the first and second bridge circuits 12, 22 for communicating using the second communication protocol.

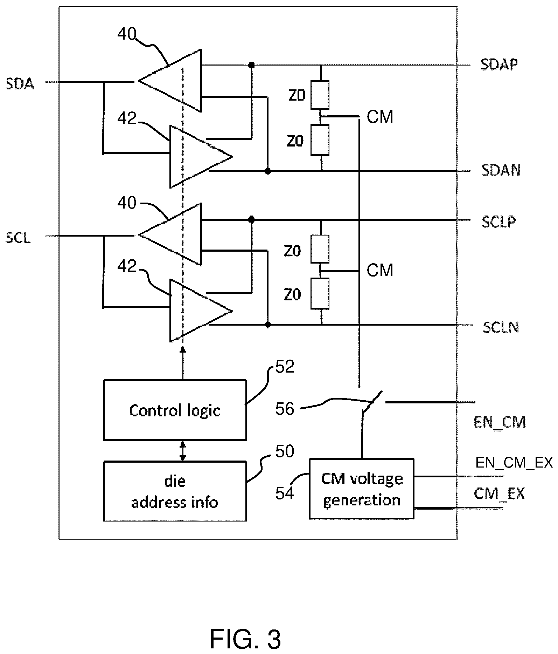

Each bridge circuit comprises a respective series pair of termination impedances (shown in Figure 3) between the wires of each differential wire pair 32, 34. The junction between the pair of termination impedances is connected to a respective common mode voltage, and these two common mode voltages may be the same and hence connected to each other. It may be possible to have a programmable common mode voltage or to modulate the common mode voltage in order to send a special message, e.g. to reset the connected devices.

This enables a four wire high-speed control interface. The interface may for example use the well-known I2C interface standard. The control interface allows programming and data read-back of the ASIC dies C3 to C5 of the sensors or actuators placed at the tip of the catheter. The speed of the catheter interface can be high (e.g. using a 20 MHz clock) due to the usage of a high speed point-to-point interface between the console unit (proximal site of the catheter) and tip using the two bridge circuits.

The bridge circuits convert signals generated by single-ended open-drain drivers forming part of the circuits C1 and C2 (such as used for a standard I2C interface) into differential small-signal swing characteristically terminated signals. Optionally, the control signals can be multiplexed with other differential catheter signals using the same system wires.

To achieve high resolution, the catheter for example operates at a high ultrasound frequency and uses a large number (e.g. more than 50, e.g. more than 100, e.g. 114) transducer elements in a small diameter catheter, e.g. below 2mm diameter such as a 3F 1 mm diameter catheter. The transducer elements can preferably be stimulated individually and the echo signals of these transducer elements can be received and processed individually.

In one design, there may be six ASIC dies integrated in the tip of the catheter. One ASIC die provides the interface with the system (console) and five ASIC dies provide the interface with the transducer elements. Thus, one ASIC functions as a local master ASIC and five others function as local slave ASICs. Note that all ASIC dies in the tip are slave ASICs in the communication protocol with the console.

The ASIC dies need programming, for example to set analogue parameters such as gain and bandwidth and to indicate which transducer element(s) must be stimulated for the next transmit event and which transducer element(s) must be selected for receive and amplification.

Figure 2 shows the system of Figure 1 in more detail for an ultrasound system. The circuits C3 to C5 comprise pulser circuits, amplifiers and selection circuitry. The ultrasound transducers are shown as a set of sub-arrays US1 to US3 together forming an overall transducer array. The ultrasound transducers are for example controlled as a synthetic aperture phased array, and they are for example formed around an annulus around the catheter tip, facing outwardly.

The first protocol is for example the I2C (2-wire) interface, used by the circuits C1 to C5. This is an attractive catheter control interface as it is well-known and as it requires only two control wires for bi-directional communication independent of the number of connected devices. However, the interface is too slow due to the usage of open-drain circuitry and pull-up resistors, in particular when heavily loaded. Programming speed can be increased by using low-value pull up resistors but this is at the expense of high power dissipation, which is not acceptable in the catheter tip.

The bridge circuits convert the single-ended e.g. I2C signals of the first protocol into small-amplitude characteristically terminated signals or vice versa. On the single-ended side of the bridge circuit, multiple I2C compatible devices / ASIC dies can be connected as commonly used by an I2C interface. On the differential side, the bridge circuits implement a high-speed characteristically terminated point-to-point interface.

Due to the small ASIC die and interconnect dimensions at the tip, the (parasitic) load on the single-ended I2C nodes is relatively low and therefore, the speed can be high even when using relatively large pull-up resistors.

The data rate is the same for the first and second communication protocols.

The length of the interconnect at the tip is for example less than 10 cm and therefore the local interconnect does not suffer from transmission line effects and can be modelled by a lumped capacitive load. Due to the short distances, the capacitive load of the open drain output at the tip can remain low as well. For example, if a pull-up resistor of 5 kΩ is used with a total capacitive load of 5 pF, a speed of 20 MHz or higher (τ = RC = 25 ns) is possible without impedance matching. If the capacitive load is higher such as 25 pF (for a longer interconnect) a smaller pull-up resistor of e.g. 1 kΩ could then be used.

The situation is different for the long catheter wires (e.g. 3m long). Transmission line effects will happen as the catheter wire length is greater than 0.1 times the wavelength of the relevant frequencies, e.g. the ones related to the clock and data transitions. In such cases, electromagnetic reflections will happen if not characteristically terminated. The characteristic termination minimizes signal reflections in order to guarantee reliable communication.

Figure 3 shows a block diagram of the bridge circuit functionality. In Figure 3, the single-ended signal names are identical to the signal names used in the I2C standard.

For completeness, a brief outline of the I2C standard will now be presented. The I2C protocol allows devices to communicate directly with each other via a simple bi-directional 2-wire bus, plus power and ground. A device connects to each of the two communications wires on the bus, which are a serial data line (SDA) for the communication of data, and a serial clock line (SCL) for the control and synchronization of the communication of data between the devices.

The output of an I2C compliant device is configured as an open-collector/open-drain, and one or more pull-up resistors maintain a soft logic high value on the bus while the bus is in a quiescent state. When a device desires access to the bus, the device pulls the bus to a logic low value, via the open-collector/open-drain device that is placed in a conductive state to ground potential.

A device that is connected to an I2C bus is identifiable by an address, and can operate as either a transmitter or a receiver, or both. Data transfers are effected using a master-slave communication protocol. A master is a device that initiates a data transfer and generates the clock signals to permit the transfer; any device that is addressed is considered a slave for this transfer. The data transfer can be initiated by a master to either transmit data to the slave (a write command), or to request data from the slave (a read command). For example, an output device such as an EEPROM memory for non-volatile storage is typically not able to initiate a data transfer, and therefore would be configured to only operate as a slave device. A sensor on the other hand, may be configured to operate as either a master or a slave, as the situation demands.

In the quiescent state, both the SDA and SCL bus lines are in the logic-high state. A master initiates a data transfer by asserting a transition to a logic-low state on the SDA line while the SCL line is high; this is termed a START condition. Thereafter, the master toggles the SCL line to control the synchronization of the data transfer; data value changes occur on the SDA line when the SCL clock is low, and the state of the SDA line is considered valid only when the SCL clock is high.

The data transfer for example comprises an address transmitted by the host (i.e. the device initiating the communication), nominally seven bits, followed by a read/write-not indicator. After transmitting the address and the direction of data transfer, the host releases the SDA line, allowing it to rise to a logic-high level. If a slave device recognizes its address, the slave device transmits an acknowledge signal (ACK) by pulling the SDA line low. The absence of a low signal when the host releases the SDA line, therefore, indicates a non- acknowledgement (NAK). If the address is acknowledged, via a low at SDA, the transmitting device transmits the data.

If the direction of data transfer is a "read" relative to the host, then the slave device is the transmitting device; if the direction is a "write" relative to the host, then the master device is the transmitting device. The transmitting device releases control of the SDA line, and the receiving device acknowledges the receipt of the data by asserting a logic-low value on the SDA line. If the data is acknowledged, the transmitter sends additional data.

This process continues until the entirety of the data is communicated, or until a transmitted data item is not-acknowledged. The master can subsequently reassert a START signal, and repeat the process above, or, can assert a STOP signal to terminate this data-transfer session. This STOP signal is a low-to-high transition on the SDA line while the SCL clock is high. Thereafter, any device may assume control of the bus as a master by asserting a high-to-low transition on the SDA line, as above.

The differential signal names (for the second protocol) are called SDAP/SDAN and SCLP/SCLN (i.e. positive SDA and negative SDA, and positive SCL and negative SCL). The bridge circuit function can however be used for other half-duplex wire interface standards as well.

Each bridge circuit comprises a respective pair of back-to-back differential amplifier circuits 40, 42 between one of the wires (SDA or SCL) for communicating using the first communication protocol and an associated one of the differential wire pairs. The bridge circuit comprises a memory 50 for storing die address information and control logic 52.

The first differential amplifiers 40 of each pair have a differential input at the side of the second communication protocol and a single-ended output at the side of the first communication protocol. The second differential amplifiers 42 of each pair have a single ended input at the side of the first communication protocol and a differential output at the side of the second communication protocol. In each pair, the single-ended input and output are connected together, and the differential input and output are connected together.

There are thus two amplifier types. A first type 40 translates a differential input signal into a single-ended output signal. A second type 42 translates a single-ended input signal into a differential output signal, The differential output of the second type 42 is connected with the differential input of the fist type.

The amplifiers behave as voltage sources. The output impedance Z0 for the amplifiers 42 may be situated inside the amplifier as well as the common mode connection.

The control logic 52 controls the operation of the amplifiers.

Examples for the voltages applied to the data lines in the circuit of Figure 3 are given below:
If SDA = + 5V => SDAP = SDAN = CM = 1 V.

If SDA=0, and an odd bit is driven from the console unit side:
=> SDAP = 1 + 0.5V
=> SDAN = 1 - 0.5V

If SDA=0, and an even bit is driven from the console unit side:
=> SDAP = 1 - 0.5V
=> SDAN= 1 + 0.5V

If SDA=0, and an odd bit is driven from catheter tip side (note that the common mode voltage may still be driven from proximal side):
=> SDAP = 1 + 0.1V
=> SDAN= 1 - 0.1V

If SDA=0, and an even bit is driven from the catheter tip side (again the common mode voltage (CM may still be driven from proximal side)
=> SDAP = 1 - 0.1V
=> SDAN = 1 + 0.1V

By way of example, it is assumed that a data pattern 10101010 is to be transmitted from the console unit to the tip, the following voltages may be generated:
SDAP: 1, 1.5, 1, 0.5, 1, 1.5, 1, 0.5
SDAN: 1, 0.5, 1, 1.5, 1, 0.5, 1, 1.5

This will make sure that the average voltage on both SDAP and SDAN remains 1 V (equal to the common mode voltage).

In the I2C protocol, the clock signal is generated by the master die (assuming clock stretching is not supported).

The "I2C idle mode" is defined by SCLK = 1 but with no activity on the lines for a longer time, and the "I2C active mode" is defined by SCLK=1

Supposing it is desired to transmit: idle, idle, idle, 010101, idle, idle from the console side, the following voltages may be generated:
SCLP: 1, 1, 1, 0.5,1.5, 0.5,1.5, 0.5,1.5, 1, 1
SCLN: 1, 1, 1, 1.5,0.5, 1.5,0.5, 1.5,0.5, 1, 1

The output at the differential side is terminated by a pair of terminating impedances Z0 between the differential lines and a common mode line CM. The two common mode lines may be connected together as shown but this is not essential. A common mode voltage generation circuit 54 supplies the common mode voltage. A switch 56 connects the common mode voltage to the common mode lines.

The control signals EN_CM_EX and CM_EX enable activation of the common mode voltage function, since the function is only needed at one side of the communication link, but identical bridge circuits may be used.

When EN_CM_EX = 1, the circuit uses the external common mode signal (CM_EX) as a reference and when EN_CM_EX=0, then the circuit generates its own reference.

CM EX and EN_CM_EX can be removed if the node CM has a pin. If an external reference voltage is used, this voltage can be applied directly to the node.

The bridge circuit continuously monitors the voltage on both sides of the bridge circuit.

The small signal differential voltage swing can be designed almost independently from the used supply voltage. If the signal swing is lower, the power dissipation will be lower due to lower currents. For example, if a voltage swing of 100 mV is used and Z0= 50Ω, the current is 2mA. If the voltage swing is 1 V, the current is 20 mA. However, it is likely that the used supply voltage at the console is higher than the used supply voltage at the tip.

The differential voltages applied to the differentially terminated nodes may be different between the console and the tip. Different I2C supply voltages are used for this purpose.

The differential amplitude generated at the catheter tip may for example be 100 mV to limit power dissipation while the differential amplitude generate at the console may be 1V for easy detection at the catheter tip.

The common mode voltage of the differential nodes is set at one or both sides of the bridge link; typically at the console side. It also is possible to have the common mode voltage set at both sides of the link; in that case, a capacitive coupling in series with the differential interconnect wires is needed. The voltage may be generated inside the bridge circuit as shown, but it may be generated external to the bridge circuit. In this latter case, the voltage is applied to the bridge circuit via a pin on the device.

The control logic 52 is in control of the directionality of the bridge circuit. The bridge circuit contains information on the I2C -addresses of the modules that are connected to the bridge circuit; based on the signals coming from either the single-ended side or the differential side, the logic will set the direction of the link in line with the I2C-protocol.

The termination resistors Z0 typically are integrated in the bridge circuit and their value is matched with the differential characteristic impedance of the interconnect wires.

The wires are controlled with equal and opposite polarity signals, so the differential impedance or odd mode impedance is relevant. Z0 is the odd mode impedance, and 2^{∗}Z0 is the differential impedance.

The odd mode impedance (Z0o) is the impedance seen when testing the impedance of one side of a pair of lines when the other is driven in equal and opposite polarity.

The pull-up resistors needed at the single ended side typically are not integrated in the module as their value may be customized towards the number of connected I2C modules and to the load of the interconnect. At the tip, the pull up resistors may be integrated to save space.

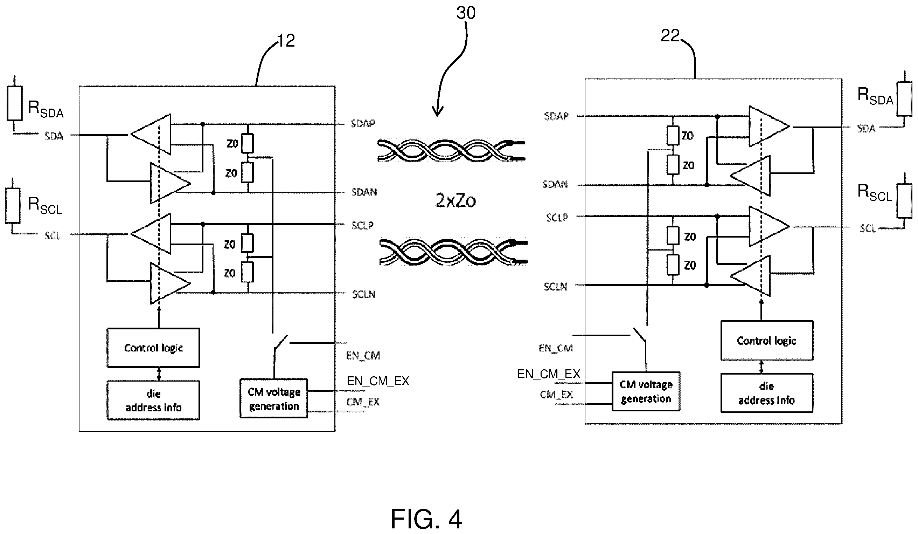

Figure 4 shows the pair of connected bridge circuits of the type shown in Figure 3. Figure 4 also shows the pull up resistors R_{SDA} for the SDA line and the pull up resistors R_{SCL} for the SCL line at the console unit and also the pull up resistors for the SDA line and for the SCL line at the tip.

To optimize the high-speed signaling characteristics and to minimize dissipation on the point-to-point interface, it is attractive to change the signaling scheme for the differential signals relative to the normal I2C-signaling scheme.

To minimize power dissipation, a differential voltage of 0V (logic "0") may be used on both SDAP/N and SCLP/N when idle. The standard I2C-signaling uses logic "1" for SDA and SCL in idling mode.

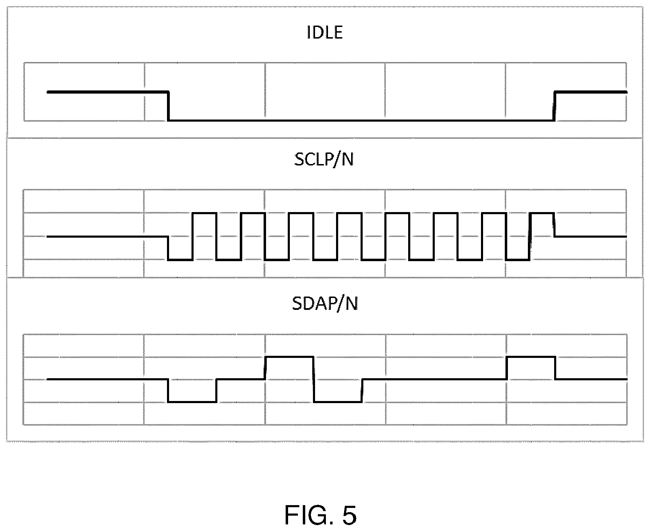

This proposed signaling scheme is shown in Figure 5. When the IDLE signal is high, the differential lines are at 0V. When the IDLE signal is low, the signaling and clock data is conveyed by the signals.

To improve the robustness of the high-speed signaling, a DC-free signaling scheme is preferred. For example, for the data signal, an inverted AMI signaling scheme (I-AMI) may be used; logic "1" is encoded as zero volt and logic "0" is encoded alternatingly as a positive and a negative voltage.

For the clock signal, a differential voltage of 0V when IDLE may be used to minimize power dissipation. When active, the clock signal may alternatingly toggle from a positive to a negative voltage. It is noted that the signal "IDLE" is not an existing signal on the interface; it is a result of the interpretation of other signals and shown for clarification only.

The ultrasound catheter is shown with two differential wire pairs. However, there are power lines as well. For example, there may be 3 power lines, VDDH (typically 30V), VDD (typically 3.3V), and GND (0V reference).

The two data communication pairs serve multiple purposes; they are used as a programming interface but also in other operation modes they also transfer the analogue echo signals of the ultrasound transducer array (from tip to console). The frequencies involved are for example up to 50 MHz. The signals also drive the trigger signal to stimulate the transducer elements (data pulses with a repetition rate of 50MHz).

The invention is for particular interest for catheters that contain multiple sensor dies and require high speed programming, for example in an advanced phased array IVUS catheter.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

## Claims

1. A catheter or guidewire system, comprising:
a console unit (10) ;
a tip (20) comprising sensors and/or actuators (US1, US2, US3); and
a catheter shaft (30) or guidewire between the console unit and the tip,
wherein the console unit comprises a first bridge circuit (12) for converting between a first communication protocol (P1) based on a two-wire single-ended open drain architecture and a second communication protocol (P2) based on two differential wire pairs, with one differential wire pair for each of the two wires of the first communication protocol,
wherein the tip comprises a second bridge circuit (22) for converting between the first and second communication protocols and for communicating with the sensors and/or actuators using the first communication protocol,
wherein the catheter shaft or guidewire comprises two differential wire pairs (32, 34) connected between the first and second bridge circuits for communicating using the second communication protocol,
and wherein each bridge circuit is terminated to match the characteristic impedance of the differential wire pairs and wherein the differential signals are superimposed on a common mode voltage.

2. The system as claimed in claim 1, where the communication between the first and second bridge circuits comprises a half duplex communication.

3. The system as claimed in claim 1 or 2 wherein each differential wire pair has a same common mode voltage (CM).,

4. The system as claimed in any preceding claim, wherein the tip comprises an ultrasound imaging device.

5. The system as claimed in claim 4, wherein the tip comprises an array of ultrasound transducer elements (UD1, US2, US3) formed as a plurality of ultrasound transducer sub-arrays, each sub-array having an associated control circuit (C3, C4, C5), wherein the second bridge circuit (22) communicates with each of the control circuits (C3, C4, C5) using the first communication protocol.

6. The system as claimed in any one of claims 1 to 5, wherein the console unit comprises a respective pull up resistor (R_{SDA} R_{SCL}) for each of the two wires for communicating using the first communication protocol, and the tip comprises a respective pull up resistor (R_{SDA} R_{SCL}) for each of the two wires for communicating using the first communication protocol.

7. The system as claimed in any one of claims 1 to 6, wherein the first communication protocol (P1) uses a pair of rail voltages with a voltage difference in the range 1.8V to 5V, and wherein the second communication protocol uses differential voltages of below 1.8V.

8. The system as claimed in claim 7, wherein the second communication protocol (P2) uses different voltage amplitudes for the tip and for the console unit.

9. The system as claimed in any one of claims 1 to 8, wherein the first communication protocol (P1) is the I²C communication protocol.

10. The system as claimed in any one of claims 1 to 9, wherein each bridge circuit (12, 22) comprises a respective pair of back-to-back amplifier circuits (40, 42) between each one of the wires for communicating using the first communication protocol and each associated one of the differential wire pairs (32, 34).

11. The system as claimed in any one of claims 1 to 10, wherein the termination of each bridge circuit comprises a respective series pair of termination impedances between the wires of each differential wire pair, wherein the junction between the pair of termination impedances is connected to the common mode voltage.

12. The system as claimed in any one of claims 1 to 11, wherein the console unit comprises a circuit (54) for setting the common mode voltage.

13. The system as claimed in any one of claims 1 to 12, wherein the second communication protocol has a data rate above 10MHz, and:
uses a zero differential voltage to encode an idling mode; and/or
uses a zero DC signaling scheme.

14. The system as claimed in any one of claims 1 to 13, wherein a one of the two differential wire pairs (34) is a clock signal wire pair and the other one of the two differential wire pairs is a data signal wire pair (32).

15. The system as claimed in any one of claims 1 to 14, wherein each bridge circuit comprises a controller (52) and a memory (50), wherein the memory stores address information of modules that are connected to the respective bridge circuit using the first communication protocol.
